Europäisches Patentamt

European Patent Office

Office européen des brevets

(19)

(11) EP 0 867 155 A2

(12) **EUROPÄISCHE PATENTANMELDUNG**

(43) Veröffentlichungstag:
30.09.1998 Patentblatt 1998/40

(51) Int. Cl.$^6$: **A61C 19/04**

(21) Anmeldenummer: 98103961.3

(22) Anmeldetag: 06.03.1998

(84) Benannte Vertragsstaaten:
**AT BE CH DE DK ES FI FR GB GR IE IT LI LU MC
NL PT SE**
Benannte Erstreckungsstaaten:
**AL LT LV MK RO SI**

(30) Priorität: 27.03.1997 CH 737/97
03.04.1997 CH 768/97
05.12.1997 CH 2822/97

(71) Anmelder:
**Ruetschi Präzisions-Technologie AG
3286 Muntelier (CH)**

(72) Erfinder:
• **Rüetschi, Paul
1421 Grandevent (CH)**
• **Ruetschi, Charles
3280 Murten (CH)**

(74) Vertreter:
**Patentanwälte
Schaad, Balass, Menzl & Partner AG
Dufourstrasse 101
Postfach
8034 Zürich (CH)**

(54) **Zahntechnische Vorrichtung und Verfahren sowie Referenzelektrode zum Feststellen des Zustandes einer Zahnkrone oder Zahnbrücke**

(57)     Die zahntechnische Vorrichtung zum Feststellen des Zustandes einer zumindest teilweise aus Metall bestehenden Zahnkrone (2) oder Zahnbrücke (2) eines Zahnes (1) umfasst eine Tast-Elektrode (3), mit welcher ein elektrischer Kontakt zur Zahnkrone (2) oder Zahnbrücke (2) herstellbar ist, einen in den Mund einlegbaren aktiven Teil (10) einer Referenz-Elektrode (9), sowie ein Spannungsmessgerät (12), das elektrisch mit der Tast-Elektrode (3) und der Referenz-Elektrode (9) verbindbar ist und deren Potential-Differenz zu messen erlaubt, wobei das Spannungsmessgerät (12) einen Eingangs-Widerstand von insbesondere mehr als $10^6 \, \Omega$ aufweist. Der aktive Teil (10) der Referenzelektrode (9) besteht insbesondere aus einem mit Silberchlorid beschichteten Silberdraht. Die Halterung (13) der Referenz-Elektorde (9) ist plastisch nach Wunsch verbiegbar und kann in den Mund eines Patienten eingehängt werden.

Fig. 1

Printed by Xerox (UK) Business Services
2.16.3/3.4

**Beschreibung**

Die Erfindung betrifft eine zahntechnische Vorrichtung sowie ein Verfahren zum Feststellen des Zustandes einer zumindest teilweise aus Metall bestehenden Zahnkrone oder Zahnbrücke. Die Erfindung betrifft weiter eine zahntechnische Referenzelektrode für die zahntechnische Vorrichtung sowie das Verfahren.

Während langer Zeit wurden zumindest teilweise aus Metall bestehende Zahnkronen oder Zahnbrücken auf Zähne montiert, ohne eventuell vorhandene Amalgamfüllungen vollständig zu entfernen. Falls Amalgam in direkten Kontakt mit beispielsweise Goldkronen oder Goldbrücken gerät, so kann am Amalgam eine beschleunigte anodische Korrosion einsetzen. Die Korrosionsprodukte des Amalgams, wie z.B. Kupfer-, Zinn-, Silber- oder Quecksilber-Ionen können auf das Zahnfleisch toxisch wirken. Unter Umständen wird soviel Quecksilber freigesetzt, dass gefährliche Quecksilber-Allergien und schwerwiegende Störungen des zentralen Nervensystems auftreten. Quecksilber-Ionen gelangen über die Blutbahn und die Blut-Gehirn-Schranke ins Gehirn, das kritische Organ für Quecksilber-Akkumulation. Aus diesem Grunde ist es angezeigt, Amalgam, welches sich unter einer zumindest teilweise aus Metall bestehenden Zahnkrone wie einer Gold-Kronen befindet, vollständig zu entfernen.

Leider ist jedoch der Nachweis für das Vorhandensein von Amalgam ohne das Zerstören und Abmontieren der Zahnkrone, bezw. der Zahnbrücke, bisher nicht möglich. Aussagekräftige Röntgenbilder können nicht gemacht werden, weil eine Gold-Krone die Röntgenstrahlen zu stark abschirmt, sodass kein Kontrast für das allfällig vorhandene Amalgam erzielt werden kann.

Es ist Aufgabe der vorliegenden Erfindung eine Vorrichtung sowie ein Verfahren zum Feststellen des Zustandes einer Zahnkrone oder Zahnbrücke vorzuschlagen, das erlaubt das Vorhandensein von Amalgam zu erkennen.

Es ist eine weitere Aufgabe der vorliegenden Erfindung eine robuste, präzise, praktische und preisgünstige zahntechnische Referenzelektrode zu schaffen, insbesondere für die Vorrichtung sowie das Verfahren zum Feststellen des Zustandes einer Zahnkrone oder Zahnbrücke.

Diese Aufgabe wrd gelöst mit einer Vorrichtung aufweisend die Merkmale von Anspruch 1. Die Unteransprüche 2 bis 8 beziehen sich auf weitere, vorteilhafte Ausgestaltungen der Vorrichtung. Die Aufgabe wird weiter gelöst mit einem Verfahren aufweisend die Merkmale von Anspruch 9. Die Unteransprüche 10 bis 13 beziehen sich auf weitere, vorteilhafte Ausgestaltungen des Verfahrens. Die Aufgabe wird weiter gelöst mit einer zahntechnischen Referenzelektrode aufweisend die Merkmale von Anspruch 14. Die Unteransprüche 15 bis 21 beziehen sich auf weitere, vorteilhafte Ausgestaltungen der Referenzelektrode.

Eine Zahnkrone oder Zahnbrücke wird allgemeiner als eine Restauration bezeichnet, wobei die Restauration üblicherweise aus Metall besteht oder zumindest teilweise aus Metall besteht, und insbesondere ein Edelmetall wie beispielsweise Gold verwendet wird. Zudem kann eine derartige metallische Restauration aus beispielsweise ästhetischen Gründen mit einem nichtmetallischen Überzug wie Keramik oder Porzellan versehen sein, so dass die gesamte Restauration zumindest teilweise aus Metall besteht, jedoch zusätzlich noch weitere nichtmetallische Anteile umfasst. Bei der Restauration von Zähnen gilt eine Krone oder Brücke aus einer Goidlegierung als die dauerhafteste, schönste, aber auch teuerste Lösung. Meist werden die sichtbaren Zahnteile mit einem Überzug aus beispielsweise Keramik oder Porzellan überzogen, was ästhetisch ansprechend ist.

Lange Zeit wurden zumindest teilweise aus Metall bestehenden Restaurationen, insbesondere Restaurationen aus Edelmetall, wie Zahnkronen oder Zahnbrücken, auf Zähne montiert, ohne die eventuell vorhandenen Amalgamfüllungen vollständig zu entfernen. Dabei wurde der Gefahr der Bildung von sogenannten Lokalelementen zu wenig Beachtung geschenkt. Zwar bildet ein Zahnzement, der zum Befestigen einer Zahnkrone oder Zahnbrücke verwendet wird, eine Isolationsschicht zwischen der Zahnkrone beziehungsweise der Zahnbrücke und der Amalgamfüllung. Eine isolierende Wirkung ist aber längerfristig oft nicht gewährleistet, zum Beispiel wenn die Zementschicht durch jahrelange Erosion teilweise zerstört wird. Durch den direkten Kontakt zwischen der insbesondere aus Gold oder einer Edelmetall-Legierung bestehenden, metallischen Zahnkrone beziehungsweise Zahnbrücke und dem Amalgam bildet sich ein Lokalelement, welches eine beschleunigte Korrosion des Amalgams sowie eventuell der Zahnkrone bewirkt.

Die Erfindung beruht auf der Erkenntnis, dass mit einer Messvorrichtung das Elektroden-Potential der Zahnkrone messbar ist, und dass aus dem gemessenen Potential beziehungsweise der gemessenen Spannung ein Hinweis auf das Vorhandensein einer anodischen Reaktionen unterhalb der Zahnkrone bzw. der Zahnbrücke herleitbar ist.

Ein Vorteil der Erfindung ist darin zu sehen, dass beim Feststellen einer anodischen Reaktion auf das Vorhandensein von Amalgam geschlossen werden kann. Ein weiterer Vorteil der Erfindung ist darin zu sehen, dass auf Grund des gemessenen Wertes des Elektroden-Potentials auf die Stärke der Korrosion geschlossen werden kann. Ein wesentlicher Vorteil der Erfindung ist darin zu sehen, dass der Nachweis von Amalgam unter einer zumindest teilweise aus Metall bestehenden Restauration wie einer Zahnkrone oder einer Zahnbrücke sowie die Stärke der Korrosion ohne ein Entfernen der Zahnkrone oder der Zahnbrücke möglich ist. Die Erfindung erlaubt eine schnelle und zerstörungsfreie Prüfung des Zustandes von Zahnkrone bzw. Zahnbrücke und ev. vorhandenem Amalgam oder allgemein des Zahnzustandes und damit einen Hinweis betreffs der Notwendigkeit einer Demontage der Zahnkrone beziehungsweise der Zahnbrücke, zwecks Restauration oder Extraktion des betreffenden, darunterliegenden Zahns. Dieser Vorteil ist von entscheidender wirtschaftlicher Bedeutung, war es doch bis anhin erforderlich zur Klärung des Zustandes einer zumindest teilweise aus Metall bestehenden Restauration wie einer Krone diese vom Zahn herabzunehmen. Insbesondere wenn sich nach einem derartigen Eingriff herausstellte, dass die Krone in einwandfreiem Zustand war, wurden unnötige Kosten und vor allem dem Patienten unnötige Unannehmlichkeiten verursacht. Ein weiterer Vorteil der Erfindung ist darin zu sehen, dass ein Zahnarzt in der Lage ist sehr schnell und zudem für den Patienten schmerzfrei zu entscheiden, ob eine erneute Restauration oder gar eine Extraktion einen Zahnes notwendig ist. Dies ermöglicht eine schnellere und kostengünstigere Behandlung. Zudem kann beim Entscheid, dass eine Extraktion erforderlich ist, auf eine vorgängige Demontage der Zahnkrone verzichtet werden.

Die anodische Reaktion kann auch auf eine Korrosion der Zahnkrone oder der Zahnbrücke selbst oder auf oxidierende organische Bestandteile hindeuten.

Die Aufgabe der Erfindung wird weiter insbesondere gelöst mit einer zahntechnische Referenzelektrode zur elektrochemischen Potentialmessung in der Mundhöhle, umfassend ein elektrochemisch aktives Teil bestehend aus einem mit Silberchlorid beschichteten Silberdraht, wobei der Silberdraht insbesondere in Form einer einfachen oder mehrfachen Schlaufe ausgestaltet ist.

In einer besonders vorteilhaften Ausführungsform umfasst die zahntechnische Referenzelektrode eine das aktive Teil bedeckende, saugfähige Umhüllung aus insbesondere elektrisch isolierenden Fasern, sowie einen plastisch oder elastisch verformbaren Stiel, in dessen Inneren eine elektrische Verbindung, welche das aktive Teil mit einer Steckbuchse verbindet, nach aussen elektrisch isoliert verlaufend angeordnet ist.

Vorteile der erfindungsgemässen zahntechnischen Referenzelektrode sind darin zu sehen, dass sie robust und präzise ist, praktisch in der Handhabung ist, preisgünstig herstellbar ist, sowie zum einmaligen Gebrauch als wegwerfbare Referenzelektrode für zahntechnische Anwendungen konzipierbar ist. Die erfindungsgemässe Referenzelektrode erlaubt, elektrochemische bzw. galvanische Potentiale von zumindest teilweise aus Metall bestehenden Restaurationen in der Mundhöhle schnell, präzise, mühelos und preisgünstig zu ermitteln. Die gemessenen Potentiale können einem Arzt zusätzliche Informationen über den Zustand von zumindest teilweise aus Metall bestehenden Restaurationen liefern. Insbesondere ergeben die gemessenen Potentiale Hinweise auf Korrosionsvorgänge, welche im Verborgenen unter Kronen oder Brücken ablaufen. Als Folge solcher Korrosionsvorgänge können in der Mundhöhle bekanntlich Ionen der Elemente Quecksilber, Cadmium, Kupfer, Silber und Zink, usw. freigesetzt werden, welche allergische oder toxische Reaktionen auslösen können. Solche Korrosionsprodukte können über die Saliva auch in den Verdauungstrakt gelangen, und von dort in andere Teile des menschlichen Organismus gelangen, wo sie in unerwünschter Weise akkummuliert werden können.

In den nachfolgenden Ausführungsbeispielen wird das Verhalten der metallischen Zahnkrone oder Zahnbrücke an Hand einer Gold-Krone erläutert. Wenn von einer Gold-Krone gesprochen wird, ist damit auch immer eine in der Zahntechnik verwendete Edelmetell-Legierung gemeint. Es zeigen:

Fig. 1      eine schematische Ansicht einer erfindungsgemässen Vorrichtung;

Fig. 2      einen Querschnitt durch einen Zahn mit ablaufender chemischer Reaktion;

Fig. 3      eine Ansicht einer Referenzelektrode;

Fig. 3a     Teilansicht eines Schnittes durch die Referenzelektrode entlang der Linie A-A gemäss Fig. 3;

Fig. 4      eine in einen Mund eingelegte Referenzelektrode;

Tab. 1      eine Übersicht festgestellter Messresultate.

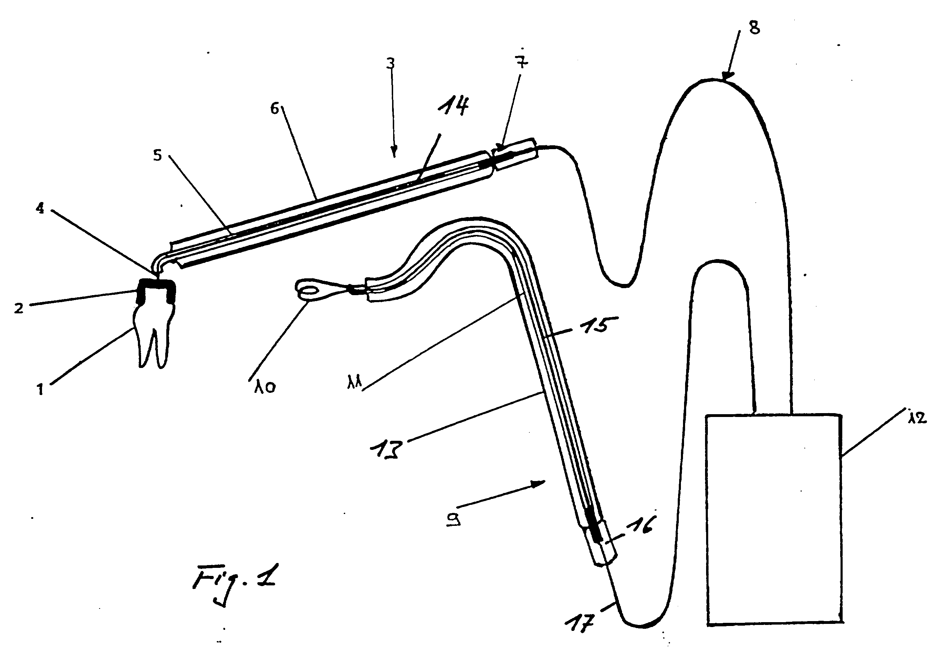

Figur 1 zeigt die erfindungsgemässe Vorrichtung. Der zu prüfende Zahn 1 mit Gold-Krone 2 oder Zahnbrücke 2 wird mit einem als Spitze ausgestalteten Kontakt 4 einer Tast-Elektrode 3 kontaktiert. Die Spitze besteht aus einer Gold-Legierung, die dem Kronen-Material ähnlich sein kann. Die Spitze kann auch nur an deren Oberfläche mit einer Schicht aus Gold oder einer Gold-Legierung versehen sein. Der Durchmesser der Spitze ist kleiner als 2 mm, am vordersten Ende vorzugsweise kleiner als 0,5 mm. Mit der Tast-Elektrode 3 wird über den Kontakt 4 eine elektrische Verbindung zur Gold-Krone 2 bzw. zur Zahnbrücke 2 hergestellt, wobei die Gold-Krone 2 bzw. die Zahnbrücke 2 die eigentliche Elektrode, d.h. die Übergangsstelle von metallischem Leiter zu einer Leitung durch Ionen darstellt.

Als Kontaktspitze 4 kann auch ein kleiner zylindrischer Stift dienen, welcher auf der zylindrischen Aussenseite mit Kunststoff isoliert ist. Die Kontaktspitze 4 ist mit einer elektrischen Ableitung 14 verbunden, die innerhalb der Tast-Elektrode 3 verlaufend angeordnet ist. Diese Ableitung 14, ausgestaltet als ein Metalldraht, ist von einer Isolation 5 umgeben. Mit Ausnahme der Kontaktspitze 4 ist die Tast-Elektrode 3 nach aussen also elektrisch hoch isoliert. Die Kontaktspitze 4 ragt weniger als 5 mm aus der Isolation 5 heraus. Ein Handgriff 6 umgibt die Isolation 5 und ist zudem derart ergonomisch ausgebildet, dass die Tast-Elektrode 3 angenehm und gut in der Hand liegt. Die Isolation 5 kann Teil des Handgriffs 6 bilden. Die elektrische Ableitung 14 ist an eine Steckbuchse 7 angeschlossen. Die Steckbuchse 7 ist über ein gegen aussen elektrisch isoliertes Anschlusskabel 8 mit einem Spannungsmessgerät 12 verbunden.

Die Vorrichtung umfasst weiter eine Referenz-Elektrode 9. Im dargestellten Ausführungsbeispiel weist die Referenz-Elektrode 9 an deren vorderen Ende ein aktives Teil 10 auf, das aus einem Silberdraht 10 besteht, welcher mit Silberchlorid beschichtet ist und derart zweckentsprechend geformt ist, dass der aktive Teil 10 in den Mund eines Patienten einlegbar ist. Der Silberdraht 10 ist mit einer elektrisch leitenden Ableitung 15 verbunden, wobei die Ableitung 15, welche als ein Metalldraht ausgestaltet ist, innerhalb der Referenz-Elektrode 9 elektrisch hoch isoliert verlaufend angeordnet ist. Die Isolation 11 umgibt die Ableitung 15. Die Isolation 11 kann in der Halterung 13 der Referenz-Elektrode 9 integriert sein. Die Referenz-Elektrode 9 ist, mit Ausnahme des aktiven Teils 10, nach aussen elektrisch hoch isoliert. Die Referenz-Elektrode 9 kann derart ausgestaltet sein, dass sie zum Einhängen in einen Unterkiefer geeignet ist, wobei der aktive Teil 10 in den Mund, vorzugsweise unter der Zunge zu liegen kommt. Der mit Silberchlorid beschichtete Silberdraht 10 wird derart im Mund plaziert, dass er keine Zahnkrone oder Zahnbrücke berührt, zum Beispiel unter der Zunge. Zum Vermeiden von Kontakt mit anderen Goldkronen oder Goldbrücken und zur Sicherstellung der Befeuchtung kann der Silberdraht 10 mit einem Überzug von saugfähiger Gaze, Tampon oder Gewebe überzogen sein. Die Gaze, der Tampon oder das Gewebe wird vor dem Einlegen des aktiven Teils 10 in den Mund beispielsweise mit einer verdünnten NaCl-Lösung getränkt. Ein derartiges Befeuchten weist den weiteren Vorteil auf, dass der aktive Teil 10 im Mund in einer definierten Elektrolytlösung angeordnet ist, was ein stabiles Potential ergibt, so dass der im Mund vorhandene pH-Wert oder die Chloridkonzentration des Speichels nur einen kleinen Einfluss auf das Potential der Referenzelektrode 9 bzw. des aktiven Teils 10 ausübt.

Für den aktiven Teil 10 der Referenz-Elektrode 9 hat sich ein Draht bzw. eine Elektrode aus Silber/Silberchlorid gut bewährt. Anstelle einer Silber/Silberchloridelektrode könnten im Prinzip auch andere Referenzelektroden 9 verwendet werden. Als Referenzelektrode 9 sind allgemein solche mit einem aktiven Teil 10 aus Metall/Metalloxid wie beispielsweise Antimon/Antimonoxid oder Quecksilber/Quecksilberoxid, oder Metall/Metallchlorid wie beispielsweise Quecksilber/Quecksilberchlorid, oder Metall/Metallsulfat geeignet. Als aktives Teil 10 der Referenzelektrode 9 eignet sich beispielsweise auch eine Glaselektrode.

Der Ableiter 15 der Referenz-Elektrode 9 ist am hinteren Ende mit einer Steckbuchse 16 versehen. Die Steckbuchse 16 ist über ein gegen aussen elektrisch isoliertes Anschlusskabel 17 mit dem Spannungsmessgerät 12 lösbar verbunden.

Das Spannungsmessgerät 12 erlaubt, die Potential-Differenz, beziehungsweise die Spannung, zwischen der Tast-Elektrode 3 und der Referenz-Elektrode 9 beziehungsweise zwischen der Kontaktspitze 4 und dem aktiven Teil 10 zu bestimmen, wobei das Spannungsmessgerät 12 beispielsweise als ein Millivoltmeter mit einem Eingangs-Widerstand von mehr als $10^6$ Ohm ausgestaltet ist. Das Spannungsmessgerät 12 weist eine nicht dargestellte Anzeigevorrichtung zur Anzeige der gemessenen Spannung oder zur Anzeige des Zustandes der Zahnkrone oder Zahnbrücke auf. Das Spannungsmessgerät kann auch elektronische Bauteile wie Spannungsteiler oder Verstärkerschaltungen und insbesondere auch eine Digitalelektronik, oder einen Mikroprozessor aufweisen.

Oft sind Zahnkronen 2 oder Zahnbrücken 2 aus ästhetischen Gründen ganz, oder nur gegen aussen, beispielsweise mit einer Porzellan- oder Keramikschicht überzogen. Auch dann kann die erfindungsgemässe Vorrichtung verwendet werden. Hierzu wird durch die Porzellan- oder Keramikschicht bis auf die Goldunterlage ein kleines Loch gebohrt, durch welches die Kontaktspitze 4 der Tast-Elektrode 3 eingeführt, und die Goldschicht kontaktiert werden kann. Das Loch ist für die Lebensdauer des Zahnes nicht von Bedeutung. Das Loch kann nach der Messung wieder zuzementiert werden.

Bei der Messung des Elektroden-Potentials fällt dem Speichel die Rolle des Elektrolyten zu. Dabei ist zu beachten, dass das mittels der Tast-Elektrode 3 gemessene Potential der Goldkrone 2 bzw. der Goldbrücke 2 vom pH, dasjenige der Referenz-Elektrode 9 bzw. des aktiven Teils 10, von der Chloridionenkonzentration abhängt. Um den Einfluss dieser Faktoren zu vermindern, kann die Mundhöhle zuvor mittels einer geeigneten Lösung gespült werden. Eine solche Lösung wäre zum Beispiel eine mit Milchsäure gepufferte, verdünnte Natriumchloridlösung.

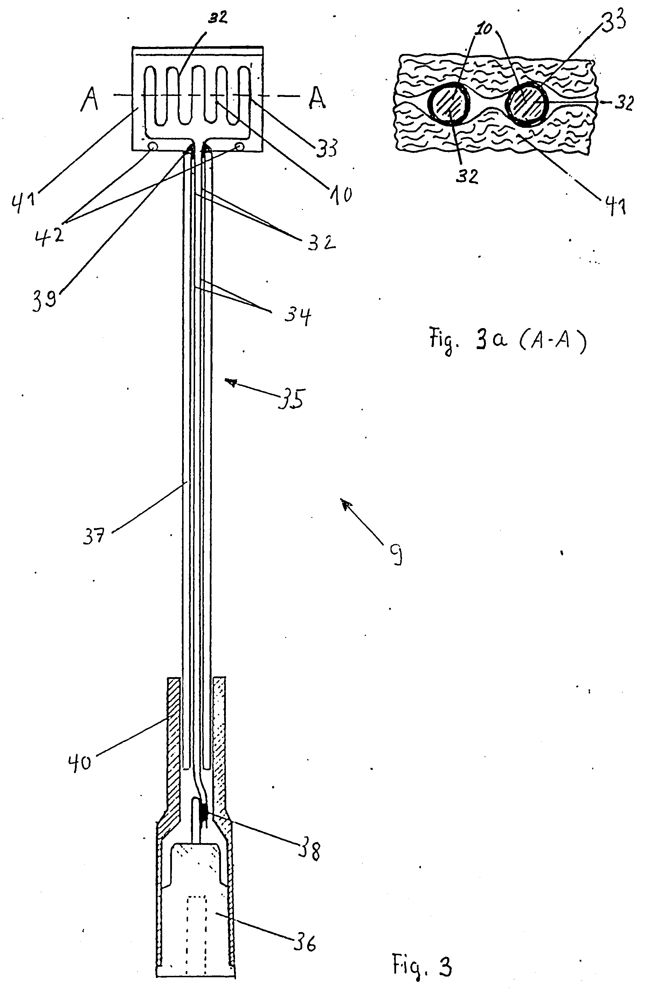

Das in Fig. 3 dargestellte Ausführungsbeispiel einer Referenzelektrode 9 weist am vorderen Ende ein aktives Teil 10 mit einem zu einer Schlaufe gebogenen Silberdraht 32 auf. Im Bereich dieses Schlaufen aufweisenden aktiven Teils 10 ist der Silberdraht 32 mit einer Schicht aus Silberchlorid 33 bedeckt. Eine elektrische Verbindung 34 verläuft innerhalb des Stiels 35 zwischen dem aktiven Teil 10 und einer Steckbuchse 36. Im Ausführungsbeispiel gemäss Fig. 3 besteht die elektrische Verbindung 34 aus einem doppelt geführten Silberdraht 32. Dieser ist über eine Lötstelle 38 mit der Steckbuchse 36 verbunden. Ueber die gesamte Verlaufslänge des Stiels 35 ist der darin verlaufende Silberdraht 32 von einem hochisolierendem Silikonschlauch 37 umgeben. Am vorderen Ende des Stiels 35 ist der Silberdraht 32 mit einem Silikonkleber 39 in den Silikonschlauch 37 eingedichtet. Ein zylinderischer Kunststoffteil 40 umgibt und isoliert nach aussen die Lötstelle 38, einen Teil der Steckbuchse 36 und den hinteren Teil des

Silikonschlauches 37. Der Kunststoffteil besteht beispielsweise aus Polyacetal-Copolymer. Die Dicke der elektrischen Verbindung 34 ist vorzugsweise so gewählt, dass sie dem Stiel 35 eine zusätzliche Festigkeit, sowie eine plastische oder elastische Verformbarkeit verleiht, so dass der Stiel 35 nach Wunsch gebogen und in den Mund eines Patienten eingehängt werden kann, vergleichbar einem Speichelsauger. Zu diesem Zweck hat der doppelt geführte Silberdraht 32 im Stiel 35 einen Durchmesser zwischen 0.6 und 1.2 mm. Im Bereich des aktiven Teils 10, welcher eine einfach oder mehrfach ausgebildete Schlaufe aus Silberdraht 32 umfasst, ist der Silberdraht 32, wie in Fig. 3a mit einem Schnitt durch zwei Silberdrähte 32 in vergrössertem Massstab dargestellt, mit einer Silberchloridschicht 33 bedeckt, welche eine Dicke von vorzugsweise zwischen 1 μm und 5 μm aufweist. Die Silberchloridschicht 33 kann beispielsweise durch Anodisieren in verdünnter Chloridlösung erzeugt werden. Der mit Silberchlorid beschichtete schlaufenförmige Teil 10 stellt die elektrochemisch aktive Oberfläche der Referenzelektrode 9 dar. Dieser Teil 10 ist mit einer saugfähigen Umhüllung 41 aus isolierenden Fasern bedeckt. Vorzugsweise besteht diese Umhüllung 41 aus einem saugfähigen Filz oder Vlies aus Baumwollfasern. Das saugfähige Vlies 41 hat vorzugsweise eine Dicke von mehr als 0.5 mm und kann pro cm$^2$ mindestens 0.1 g Wasser aufsaugen. Am Rande ist das saugfähige Vlies 41 an einzelnen Punkten 42 verklebt oder vernäht, so dass es nicht von dem als Schlaufe ausgebildeten aktiven Teil 10 abgestreift werden kann. Das saugfähige Vlies 41 umgibt die Silberdrahtschlaufe derart, dass ein direkter elektrischer Kontakt zwischen der Schlaufe und einer zumindest teilweise aus Metall bestehenden Restauration in der Mundhöhle verunmöglicht wird.

Die erfindungsgemässe Referenzelektrode 9 weist wesentliche Vorteile auf, indem sie sehr robust ist. Die erfindungsgemässe Referenzelektrode 9 enthält keine Teile aus Glas, ist schock- und bruchsicher, und zudem leicht und handlich. Die Referenzelektrode 9 weist, insbesondere auch durch die schlaufenförmige Ausbildung des elektrochemisch aktiven Teils 10, eine sehr gute Stabilität und Strombelastbarkeit auf. Der elektrochemisch aktive Teile 10 weist kein spitzes Ende auf, wodurch Stichverletzungen verunmöglicht sind. Infolge der schlaufenförmigen Ausbildung weist die Silberchloridschicht 33 eine gleichmässige Dicke auf, wodurch ein Abblättern vermieden wird.

Ein weiterer Vorteil der erfindungsgemässen Referenzelektrode 9 ist deren einfache Handhabung, welche dank des biegbaren Stiels, ähnlich einem Saliva-Absauger, ein Einlegen unter die Zunge erlaubt. Ein Arzt braucht daher während der Messung die Referenzelektrode 9 nicht mit der Hand zu halten. Ein weiterer praktischer Vorteil ist, dass die Referenzelektroden 9 vor dem Gebrauch in trockenem Zustand monatelang gelagert werden können. Vorzugsweise werden sie in versiegelten Kunststofffolien-Verpackungen aufbewahrt.

Unmittelbar vor dem Gebrauch wird die Referenzelektrode 9 der Verpackung entnommen und mit dem Anschlusskabel 17 zum Voltmeter 12 verbunden. Der aktive Teil 10, welcher mit der saugfähigen Umhüllung 41 bedeckt ist, wird sodann für einige Sekunden in eine verdünnte, kalibrierte Standardlösung mit Choridionen getaucht. Die Chloridionen-Lösung sollte eine Konzentration aufweisen, welche wenigstens derjenigen von natürlicher Saliva entspricht oder sie übersteigt. In praktischen Versuchen hat sich eine Konzentration von 0.05 Mol/Liter als geeignet erwiesen. Beim Eintauchen sättigt sich die saugfähige Umhüllung 41 mit Elektrolytlösung. Dadurch wird eine gleichmässige Benetzung der Silberchloridschicht 33 gewährleistet. Die saugfähige Umhüllung 41, beispielsweise ein Vlies aus Baumwollfasern, saugt so viel Elektrolyt auf, dass die Konzentration während der kurzen Zeit (einige Sekunden) der Messung auf der aktiven Oberfläche konstant bleibt, und genau definiert ist. Die mit Elektrolyt getränkte Umhüllung 41 stellt auch eine ausgedehnte elektrolytische Kontaktfläche zur Saliva der Mundhöhle sicher. Da die saugfähige Umhüllung elektrisch nichtleitend ist, wird ein direkter elektrischer Kontakt zwischen der Silberdrahtschlaufe des aktiven Teils 10 und einer zumindest teilweise aus Metall bestehenden Restauration verunmöglicht. Die erfindungsgemässe Referenzelektrode 9 erlaubt einen ausserordentlich niederohmigen Messkreis.

Ein weiterer Vorteil der Ausführung gemäss Fig. 3 ist der grosse Abstand zwischen der mit Elektrolyt getränkten Umhüllung 41 und der Lötstelle 38 zur Steckbuchse 36. Dies bietet zusätzliche Sicherheit, dass beim Gebrauch kein Elektrolyt oder keine Saliva zur Lötstelle 38 gelangen kann. Sollte der Elektrolyt zur Lötstelle gelangen, so könnte dies dort elektrochemische Reaktionen bewirken, welche die Potentialmessungen verfälschen könnten.

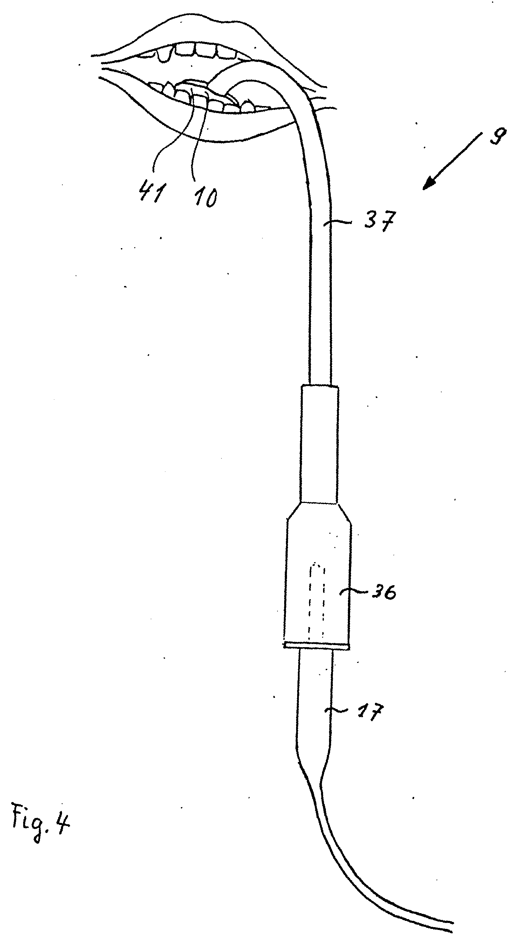

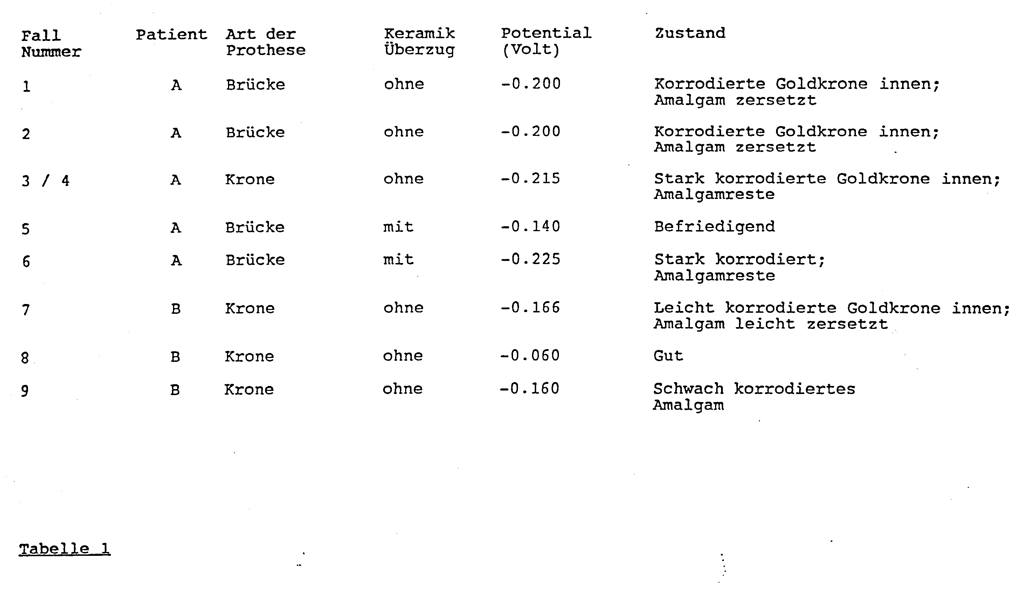

Ein weiterer Vorteil der erfindungsgemässen Referenzelektrode 9 besteht darin, dass sie serienmässig sehr preisgünstig fabriziert werden kann. Sie eignet sich deshalb für einen einmaligen Gebrauch. Sie kann in trockenem Zustand verpackt und gelagert werden und ist im Bedarfsfall ausserordentlich schnell gebrauchsfertig. Es genügt, den elektrochemisch aktiven Teil 10 mit Umhüllung 41 für einige Augenblicke in Elektrolytlösung zu tauchen, welche zusammen mit der Referenzelektrode 9 mitgeliefert wird. Zur Messung des Potentials von zumindest teilweise aus Metall bestehenden Restaurationen wird der aktive Teil 10 mit Umhüllung 41 in den Mund, vorzugsweise unter die Zunge gelegt, und der Stiel 35 in gewünschter Weise gebogen, wie dies in Fig. 4 dargestellt ist. Unter Verwendung einer Tastelektrode 3 mit Edelmetallspitze 4, mit welcher die zu untersuchende zumindest teilweise aus Metall bestehenden Restauration kontaktiert wird, kann das elektrochemische Potential der Restauration gemessen werden. Die Messung benötigt nur wenige Sekunden.

In Tabelle I sind experimentelle Resultate zusammengestellt, welche mittels der Vorrichtung gemäss Fig. 1 gemessen wurden. Für die Messungen an Zahnbrükken, welche mit einem Porzellanüberzug versehen waren, wurde durch diesen ein kleines Loch bis zur Goldunterlage gebohrt. Die gemessenen Potentiale lagen im Bereich zwischen 0 und -0.250 Volt (gegen eine Silber/Silberchlorid Referenz-Elektrode in 0.05 molarer Chloridlösung). Nach dem Messen des Potentials wurde die betreffende Zahnbrücke 2 oder Zahnkrone 2 abmontiert und der Zustand des darunterliegenden Zahns 1 analysiert. Überreste von Amalgam, sowie die Innenseite der Krone, bzw. Brücke wurden mittels Mikro-Röntgenfluoreszenz-Spektroskopie untersucht. Zwischen dem gemessenen Potential und dem Zustand der Zahnkrone 2 oder Zahnbrücke 2 bzw. der von einer Zahnkrone 2 oder Zahnbrücke 2 zumindest teilweise überdeckten Zahnfüllung 21 aus Amalgam besteht eine eindeutige Korrelation. Bei Potentialen, welche negativer waren als -0.150 Volt wurden Korrosionserscheinungen festgestellt. Im Gegensatz hierzu war der Zustand des Zahnes bzw. der Zahnkrone oder Zahnbrücke bzw. der Zahnfüllungen aus Amalgam bei Potentialen zwischen 0 und -0.150 Volt zufriedenstellend oder einwandfrei.

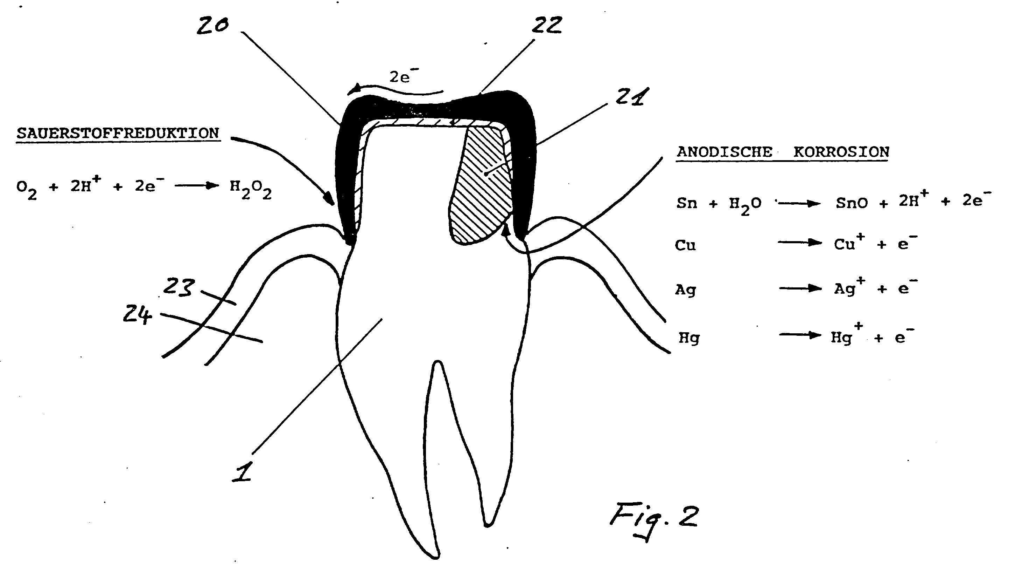

Die potentialbestimmenden elektrochemischen Reaktionen werden an Hand von Fig. 2 erklärt. Fig. 2 zeigt einen Querschnitt durch einen Zahn 1 mit einer Goldkrone 20, einer Amalgamfüllung 21 und einer zwischen der Amalgamfüllung 21, beziehungsweise dem Zahn 1, und der Goldkrone 20 verlaufenden Schicht Zahnzement 22. Der Zahn 1 ist im Knochen 24 eingewachsen und von Zahnfleisch 23 umgeben.

Die mit der Vorrichtung gemäss Fig. 1 gemessenen, und in Tabelle 1 aufgelisteten Potentiale können in vereinfachter Form wie folgt erklärt werden:

Es ist anzunehmen, dass das Elektroden-Potential von Goldkronen und Goldbrücken wesentlich durch eine elektrochemische Reaktion bestimmt wird, welche als Reduktion von Sauerstoff zu Wasserstoffperoxid beschrieben werden kann. Gold ist nämlich ein eher schlechter Katalysator für die Zersetzung von Peroxid und deshalb ist die Reduktion bis zur Endstufe Wasser unterdrückt.

$$O_2 + 2H^+ + 2e^- \rightleftarrows H_2O_2$$

Das theoretische Elektroden-Potential (gegen eine Standard-Wasserstoffelektrode) dieser Reaktion ist bei gleichen Aktivitäten von $O_2$ und $H_2O_2$ gegeben durch

$$E = 0.682 - 0.059 \text{ pH}$$

Als Elektrolyt dient die Saliva der Mundhöhle oder die Körperflüssigkeit des Zahnfleisches. Bei einem pH von 6 errechnet sich dann zum Beispiel ein Potential von E = 0.328 Volt.

Das Potential einer Silber/Silberchlorid Elektrode, anderseits, ist bestimmt durch den Elektroden-Prozess

$$AgCl + e^- \rightleftarrows Ag + Cl^-$$

und das theoretische Elektrodenpotential (gegen eine Standard-Wasserstoffelektrode beträgt

$$E = 0.222 - 0.059 \log (Cl^-)$$

wobei $(Cl^-)$ die Konzentration (genauer die Aktivität) der Chloridionen im Elektrolyt bezeichnet. Für eine 0.01 molare NaCl Lösung errechnet man somit ein Potential von 0.340 Volt.

Man sieht aus dem Vorhergehenden, dass das Potential einer Goldelektrode und dasjenige einer Ag/AgCl Referenz-Elektrode unter diesen Bedingungen sehr nahe beieinander liegen. Die theoretische Spannungsdifferenz zwischen diesen beiden Elektroden beträgt nur -0.012 Volt. Die Goldelektrode ist negativ gegen die Ag/AgCl Referenz-Elektrode.

Diese theoretische Berechnung ist in guter Übereinstimmung mit dem experimentellen Befund. Für eine Goldelektrode welche in luftgesättigte 0,01 molare Natriumchloridlösung eintauchte, beobachteten wir beim Laborversuch, gegen eine Ag/AgCl Referenz-Elektrode in derselben Lösung eine Spannungsdifferenz von -0.020 Volt. Für Goldlegierungen wie sie in der Zahntechnik üblich sind, betrugen die gemessenen Werte -0.030 bis -0.050 Volt. Solche Werte würde man theoretisch auch in vivo, d.h. in der Saliva der Mundhöhle erwarten. Die experimentellen Resultate bestätigen dies, unter der Voraussetzung, dass die Sauerstoffreduktion das Potential weitgehend bestimmt.

Indessen wurde erkannt, dass das Potential von Goldkronen und -Brücken durch anodische elektrochemische Prozesse, welche (im Verborgenen) unter Kronen oder Brücken ablaufen, beeinflusst werden kann. Ein derartiger anodischer Prozess ist z.B. die anodische Oxidation (Korrosion) von Amalgam. Andere anodische Prozesse sind die anodische Oxidation der Innenfläche der Goldkrone, bezw. Brücke oder die anodische Oxidation von organischem Material (Speisereste oder Zersetzungsprodukte des Zahnfleisches). Alle diese anodischen Reaktionen produzieren Elektronen, welche ihrerseits zur Sauerstoffreduktion dienen. Im Falle von Amalgamkorrosion kann man die anodischen Prozesse wie folgt beschreiben

$$Sn \text{ (im Amalgam)} + H_2O \rightarrow SnO + 2H^+ + 2e^-$$

$$Cu \text{ (im Amalgam)} \rightarrow Cu^+ + e^-$$

$$Hg \text{ (im Amalgam)} \rightarrow Hg_2^{2+} + 2e^-$$

$$Ag \text{ (im Amalgam)} \rightarrow Ag^+ + e^-$$

Die entstehenden Elektronen fliessen durch die Goldkrone, bezw. Brücke zum Ort der Sauerstoffreduktion und werden dort verbraucht:

$$O_2 + 2\,H^+ + 2\,e^- \rightarrow H_2O_2$$

Durch die obigen gekoppelten Reaktionen wird der Goldkrone, bezw. Brücke ein sogenanntes Mischpotential aufgedrückt. Durch den (internen) Stromfluss wird das Potential der Gold-Krone (-Sauerstoffelektrode) auf einen niedrigeren Wert gedrückt. Dieser Mechanismus erklärt, wieso beim Vorhandensein von anodischen Reaktionen das Potential einer Goldkrone, bezw. Brücke tiefer liegt. (Siehe Fig. 2).

Wie aus den experimentellen Resultaten der Tabelle 1 hervorgeht, weisen Potentiale, welche negativer liegen als -0.150 Volt auf das Vorhandensein von korrodierendem Amalgam hin, wenn als aktives Teil 10 eine Silber/Silberchlorid Elektrode bezw. eine Silber/Silberchlorid Referenzelektrode 9 verwendet wird. Entsprechend der elektrochemischen Spannungsreihe weist eine Referenzelektrode 9 aus einem anderen Material, z.B. aus Antimon/Antimonoxid einen anderen charakteristischen Spannungswert auf, bei dessen Unterschreiten auf eine anodische Reaktion zu schliessen ist.

Die erfindungsgemässe Vorrichtung sowie das Verfahren erlaubt daher eine Diagnose des Zahnzustandes unter einer Zahnbrücke der Zahnkrone. Zeigt die Potentialmessung mittels einer Silber/Silberchlorid Referenzelektrode einen Wert, welcher negativer liegt als -0.150 Volt, sollte die Zahnkrone, bezw. Zahnbrücke zwecks Sanierung des darunterliegenden Zahns entfernt werden.

Für Untersuchungen an Zahnkronen, die mehr als einen Zahn überdecken oder für Zahnbrücken, die Zähne miteinander metallisch leitend verbinden, muss berücksichtigt werden, dass das gemessene Potential ein mittlerer Wert für die betroffenen Zähne ist.

Deutet der Messwert doch auf ein Vorhandensein von Amalgam hin, so sollten zuerst die metallisch leitend verbundenen Zähne bezw. Kronen getrennt werden, so dass die Zähne einzeln gemessen und beurteilt werden können.

Die Messung zeigt dann, welche Zähne zuerst erneut restauriert werden sollten.

Es kann sich als vorteilhaft erweisen vor einer Messung mit der erfindungsgemässen Vorrichtung einen gelierten Elektrolyt im Bereich der zu messenden Zahnkrone 2 oder Zahnbrücke 2 und/oder im Bereich des aktiven Teils 10 der Referenzelektrode 9 aufzubringen.

**Patentansprüche**

1. Zahntechnische Vorrichtung zum Feststellen des Zustandes einer zumindest teilweise aus Metall bestehenden Zahnkrone (2) oder Zahnbrücke (2) eines Zahnes (1), umfassend eine Tast-Elektrode (3), mit welcher ein elektrischer Kontakt (4) zur Zahnkrone (2) oder Zahnbrücke (2) herstellbar ist, ein in den Mund einlegbares aktives Teil (10) einer Referenz-Elektrode (9), sowie ein Spannungsmessgerät (12), das elektrisch mit der Tast-Elektrode (3) und der Referenz-Elektrode (9) verbindbar ist und deren Potential-Differenz zu messen erlaubt.

2. Vorrichtung nach Anspruch 1, dadurch gekennzeichnet, dass die Tast-Elektrode (3) eine elektrische Kontaktstelle (4) aufweist, und dass zumindest die Oberfläche der Kontaktstelle (4) aus Gold, einer Goldlegierung oder einem ähnlichen Material wie die Zahnkrone (2) oder Zahnbrücke (2) besteht.

3. Vorrichtung nach Anspruch 2, dadurch gekennzeichnet, dass die Kontaktstelle (4) mit einer elektrischen Ableitung (14) verbunden ist, und dass die Ableitung (14) in der Tast-Elektrode (3) elektrisch isoliert verlaufend angeordnet ist.

4. Vorrichtung nach einem der vorhergehenden Ansprüche, dadurch gekennzeichnet, dass der aktive Teil (10) der Referenz-Elektrode (9) aus Metall/Metallchlorid oder Metall/Metallsulfat oder Metall/Metalloxid besteht oder als eine Glaselektrode ausgebildet ist.

5. Vorrichtung nach Anspruch 4, dadurch gekennzeichnet, dass die Referenz-Elektrode (9) einen aus Silber bestehenden aktiven Teil (10) aufweist, dessen Oberfläche mit Silberchlorid beschichtet ist.

6. Vorrichtung nach einem der vorhergehenden Ansprüche, dadurch gekennzeichnet, dass der aktive Teil (10) mit einer elektrischen Ableitung (15) verbunden ist, dass die Ableitung (15) in der Referenz-Elektrode (9) elektrisch isoliert verlaufend angeordnet ist, und dass die Referenz-Elektrode (9) insbesondere zum Einhängen in einen Unterkiefer ausgestaltet ist, wobei der aktive Teil (10) in den Mund zu liegen kommt.

7. Vorrichtung nach einem der vorhergehenden Ansprüche, dadurch gekennzeichnet, dass das Spannungsmessgerät (12) einen Eingangs-Widerstand von mehr als $10^6\ \Omega$ aufweist.

8. Vorrichtung nach einem der vorhergehenden Ansprüche, dadurch gekennzeichnet, dass der aktive Teil (10) von einem eine Flüssigkeit aufnehmenden Mittel umgeben ist, insbesodere einer Gaze, einem Tampon oder einem Gewebe.

9. Verfahren zum Feststellen des Zustandes einer zumindest teilweise aus Metall bestehenden Zahnkrone (2) oder Zahnbrücke (2) eines Zahnes (1), indem ein aktiver Teil (10) einer Referenz-Elektrode (10) in den Mund, insbesondere unter die Zunge gelegt wird, indem mit einer Tast-Elektrode (3) ein elektrischer Kontakt zur Zahnkrone (2) oder Zahnbrücke (2) hergestellt wird, und indem mit einem Spannungsmessgerät (12) die Potential-Differenz zwischen der Tast-Elektrode (3) und dem aktiven Teil (10) der Referenz-Elektrode (9) gemessen wird.

10. Verfahren nach Anspruch 9, dadurch gekennzeichnet, dass, sofern das aktive Teil (10) der Referenz-Elektrode (9) aus mit Silberchlorid beschichtetem Silber besteht, bei Spannungen negativer als -150 Millivolt auf ein Vorhandensein einer unerwünschten elektrochemischen Reaktion unterhalb der Zahnkrone (2) oder der Zahnbrücke (2) geschlossen wird.

11. Verfahren nach einem der Ansprüche 9 oder 10, dadurch gekennzeichnet, dass bei einer aus Metall bestehenden Zahnkrone (2) oder Zahnbrücke (2), welche mit einer Keramik beschichtet ist, ein Loch von insbesondere weniger als 2 mm Durchmesser durch die Keramik gebohrt wird, und dass der Kontaktstift (4) der Tast-Elektrode (3) in das Loch eingeführt wird und dabei ein elektrischer Kontakt zwischen der Tast-Elektrode (3) und der metallischen Zahnkrone (2) oder Zahnbrücke (2) hergestellt wird.

12. Verfahren nach einem der Ansprüche 9 bis 11, dadurch gekennzeichnet, dass ein gelierter Elektrolyt im Bereich der zu messenden Zahnkrone (2) oder Zahnbrücke (2) und/oder im Bereich des aktiven Teils (10) der Referenzelektrode (9) aufgebracht wird.

13. Verfahren nach einem der Ansprüche 9 bis 12, dadurch gekennzeichnet, dass der pH-Wert und die Natriumchlorid-Konzentration der Saliva vor dem Messvorgang durch eine Spülung der Mundhöhle mit einer gepufferten Natriumchloridlösung stabilisiert wird.

14. Zahntechnische Referenzelektrode (9), insbesondere für eine Vorrichtung gemäss einem der Ansprüche 1 bis 8, zur elektrochemischen Potentialmessung in der Mundhöhle, umfassend ein elektrochemisch aktives Teil (10) bestehend aus einem mit Silberchlorid (33) beschichteten Silberdraht (32), wobei der Silberdraht (32) insbesondere in Form einer einfachen oder mehrfachen Schlaufe ausgestaltet ist.

15. Zahntechnische Referenzelektrode (9) nach Anspruch 14, umfassend eine das aktive Teil (10) bedeckende, saugfähige Umhüllung (41) aus insbesondere elektrisch isolierenden Fasern, sowie einen plastisch verformbaren Stiel (35), in dessen Inneren eine elektrische Verbindung (34), welche das aktive Teil (10) mit einer Steckbuchse (36) verbindet, nach aussen elektrisch isoliert verlaufend angeordnet ist.

16. Zahntechnische Referenzelektrode (9) nach einem der Ansprüche 14 oder 15, dadurch gekennzeichnet, dass der Silberdraht (32) einen Durchmesser zwischen 0,6 mm und 1,2 mm aufweist.

17. Zahntechnische Referenzelektrode (9) nach einem der Ansprüche 14 bis 16, dadurch gekennzeichnet, dass die im Inneren des Stiels (35) verlaufende elektrische Verbindung (34) aus Silberdraht (32) besteht, welcher am einen hinteren Ende des Stiels (35) mit der Steckbuchse (36) verbunden und insbesondere angelötet ist, und dass der Silberdraht (32) im Stiel (35) insbesondere doppelt geführt ist.

18. Zahntechnische Referenzelektrode (9) nach einem der Ansprüche 14 bis 17, dadurch gekennzeichnet, dass die Silberchloridschicht (33) auf dem elektrochemisch aktiven Teil (10) eine Dicke zwischen 1 $\mu$m und 5 $\mu$m aufweist und insbesondere mittels anodischer Oxidation in verdünnter Chloridlösung erzeugt ist.

19. Zahntechnische Referenzelektrode (9) nach einem der Ansprüche 14 bis 18, dadurch gekennzeichnet, dass der Stiel (35) einen Silikonschlauch (37) als gegen aussen wirkenden Isolator aufweist.

20. Zahntechnische Referenzelektrode (9) nach einem der Ansprüche 14 bis 19, dadurch gekennzeichnet, dass der Silberdraht (32) an einem vorderen Ende des Stiels (35) mittels eines Silikonklebers in die elektrische Isolation des Stiels (35) eingedichtet ist.

21. Zahntechnische Referenzelektrode (9) nach einem der Ansprüche 14 bis 20, dadurch gekennzeichnet, dass die saugfähige Umhüllung (41) aus einem Filz aus Baumwollfasern besteht, dass dieser Filz insbesondere eine Dicke von mehr als 0.5 mm aufweist, und dass dieser Filz insbesondere pro cm$^2$ mehr als 0,1 g Wasser aufsaugen kann.

22. Zahntechnische Vorrichtung nach einem der Ansprüche 1 bis 8 betrieben mit einem Verfahren nach einem der Ansprüche 9 bis 13.

Fig. 1

SAUERSTOFFREDUKTION

$$O_2 + 2H^+ + 2e^- \longrightarrow H_2O_2$$

ANODISCHE KORROSION

$$Sn + H_2O \longrightarrow SnO + 2H^+ + 2e^-$$

$$Cu \longrightarrow Cu^+ + e^-$$

$$Ag \longrightarrow Ag^+ + e^-$$

$$Hg \longrightarrow Hg^+ + e^-$$

Fig. 2

EP 0 867 155 A2

Fig. 3a (A-A)

Fig. 3

Fig. 4

| Fall Nummer | Patient | Art der Prothese | Keramik Überzug | Potential (Volt) | Zustand |
|---|---|---|---|---|---|
| 1 | A | Brücke | ohne | -0.200 | Korrodierte Goldkrone innen; Amalgam zersetzt |
| 2 | A | Brücke | ohne | -0.200 | Korrodierte Goldkrone innen; Amalgam zersetzt |
| 3 / 4 | A | Krone | ohne | -0.215 | Stark korrodierte Goldkrone innen; Amalgamreste |
| 5 | A | Brücke | mit | -0.140 | Befriedigend |
| 6 | A | Brücke | mit | -0.225 | Stark korrodiert; Amalgamreste |
| 7 | B | Krone | ohne | -0.166 | Leicht korrodierte Goldkrone innen; Amalgam leicht zersetzt |
| 8 | B | Krone | ohne | -0.060 | Gut |
| 9 | B | Krone | ohne | -0.160 | Schwach korrodiertes Amalgam |

Tabelle 1

EP 0 867 155 A2